(19) Europäisches Patentamt
European Patent Office
Office européen des brevets

(11) **EP 2 728 549 B1**

(12) **EUROPEAN PATENT SPECIFICATION**

(45) Date of publication and mention
of the grant of the patent:
**12.12.2018 Bulletin 2018/50**

(51) Int Cl.:
**_G06T 7/00_** _(2017.01)_     **_A61B 3/10_** _(2006.01)_
**_G01B 9/02_** _(2006.01)_

(21) Application number: **13190930.1**

(22) Date of filing: **30.10.2013**

(54) **Image processing apparatus and image processing method**

Bildverarbeitungsvorrichtung und Bildverarbeitungsverfahren

Appareil et procédé de traitement d'images

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.10.2012  JP 2012239273
31.07.2013  JP 2013159180**

(43) Date of publication of application:
**07.05.2014  Bulletin 2014/19**

(73) Proprietor: **CANON KABUSHIKI KAISHA
Ohta-ku
Tokyo 146-8501 (JP)**

(72) Inventors:
• **Iwase, Yoshihiko**
**Ohta-ku, Tokyo (JP)**
• **Sato, Makoto**
**Ohta-ku, Tokyo (JP)**

(74) Representative: **Hitching, Peter Matthew et al
Canon Europe Ltd
European Patent Department
3 The Square
Stockley Park
Uxbridge Middlesex UB11 1ET (GB)**

(56) References cited:
**EP-A1- 2 574 273        EP-A1- 2 617 351
US-A1- 2007 115 481    US-A1- 2010 166 293**

EP 2 728 549 B1

**Description**

BACKGROUND OF THE INVENTION

Field of the Invention

The present invention relates to an image processing apparatus and an image processing method for processing an image of a subject.

Description of the Related Art

An optical coherence tomography (OCT) using a multiple-wavelength lightwave interference can acquire a high resolution tomographic image of a sample (fundus in particular).
In recent years, an ophthalmologic OCT apparatus can acquire not only a normal OCT image in which the shape of a fundus tissue is captured but also a polarization-sensitive OCT image captured using a polarization parameter (retardation and orientation), which is one of optical characteristics of the fundus tissue.
The polarization-sensitive OCT can configure the polarization-sensitive OCT image using the polarization parameter, and can perform distinction and segmentation of the fundus tissue. The polarization-sensitive OCT uses light modulated into circularly polarized light as measuring beam for observing the sample to detect interfering light split as two orthogonal linear polarization and generate the polarization-sensitive OCT image (refer to International Patent Application WO2010/122118A1).
In general, an atrophic region of a retinal pigment epithelium (RPE) (a discontinuous region in a predetermined layer) can be recognized from a polarization-sensitive OCT image of a subject's eye in disorder. EP 2 617 351 A1 and EP 2 574 273 A1 describe such an apparatus and method.

SUMMARY OF THE INVENTION

The present invention is directed to an image processing apparatus and an image processing method capable of providing a user with information effective for diagnosis depending on the state of a atrophic region.
According to a first aspect of the present invention, there is provided an image processing apparatus as specified in claims 1 to 7. According to a second aspect of the present invention, there is provided a method for controlling an image processing apparatus as specified in clams 8 to 14. According to a third aspect of the present invention, there is provided a computer-executable program for causing a computer to execute each step of the image processing method as specified in clam 15.
Further features of the present invention will become apparent from the following detailed description of exemplary embodiments with reference to the attached drawings.

BRIEF DESCRIPTION OF THE DRAWINGS

Fig. 1 is a schematic diagram illustrating an overall configuration of an image processing apparatus according to an exemplary embodiment.
Figs. 2A to 2E are examples of images generated by a signal processing unit 190.
Fig. 3 is a flowchart illustrating a processing flow according to the present exemplary embodiment.
Fig. 4 is an display example illustrating a display screen of a display unit of the image processing apparatus according to the present exemplary embodiment.
Figs. 5A and 5B are diagrams illustrating a tomographic image and a depolarized area according to present exemplary embodiment.
Figs. 6A to 6D are diagrams illustrating tomographic images of a lesion and depolarized areas according to the present exemplary embodiment.
Figs . 7A to 7E are diagrams illustrating fundus images of a lesion according to the present exemplary embodiment.
Fig. 8 is a display example of a display screen on a display unit of the image processing apparatus according to the present exemplary embodiment.
Figs. 9A to 9D are diagrams illustrating tomographic images of a lesion and depolarized areas according to the present exemplary embodiment.

DESCRIPTION OF THE EMBODIMENTS

An imaging apparatus according to the present invention is applicable to subjects such as a subject's eye, a skin, and internal organs. The imaging apparatus according to the present invention is an ophthalmologic apparatus and an endoscope, for example. The following describes in detail an ophthalmologic apparatus according to the present exemplary embodiment as an example of the present invention with reference to the attached drawings.

<Overall configuration of the apparatus>

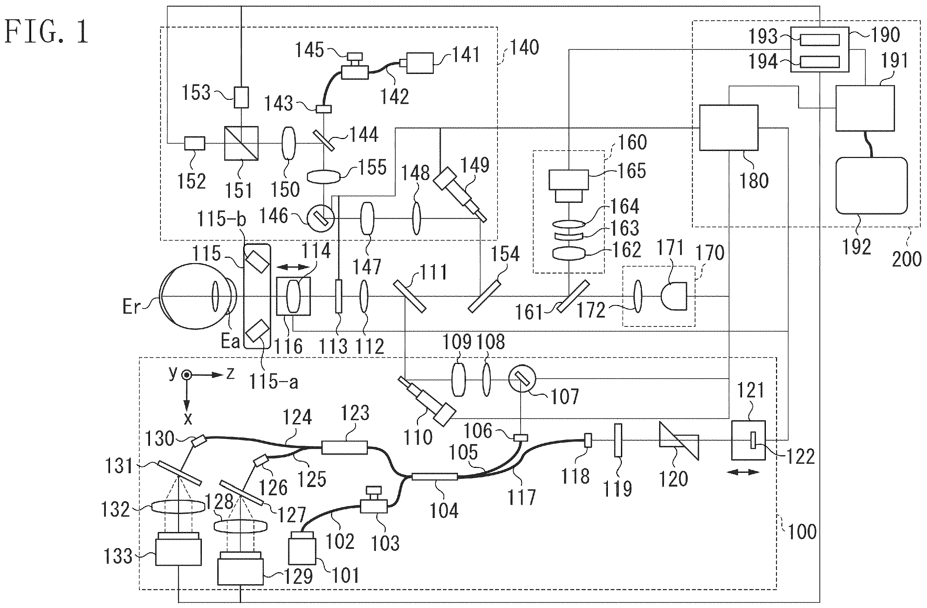

Fig. 1 is a schematic diagram illustrating an overall configuration of an "ophthalmologic apparatus" which is an example of an imaging apparatus in the present exemplary embodiment. At least a part of a signal processing unit 190 described below can be regarded as an "image processing apparatus". In this case, the entire "ophthalmologic apparatus" can be regarded as an "ophthalmologic system", or the entire "imaging apparatus" can be regarded as an "imaging system".

The ophthalmologic apparatus includes an polarization sensitive OCT 100 (hereinafter referred to as PS-OCT), a polarization sensitive scanning laser ophothalmoscope 140 (hereinafter referred to as PS-SLO), an anterior eye imaging unit 160, an internal fixation lamp 170, and a control unit 200.

Alignment of the ophthalmologic apparatus is performed using an image at an anterior eye portion of the subject observed by the anterior eye imaging unit 160 with the internal fixation lamp 170 turned on and the subject's eye caused to gaze thereat. After the alignment is completed, the PS-OCT 100 and the PS-SLO 140 perform imaging of the fundus.

<Configuration of OCT 100>

The configuration of the OCT 100 is described.

A light source 101 is a super luminescent diode (SLD) light source low in coherence and emits light with a center wavelength of 850 nm and a band width of 50 nm, for example. Although the SLD is used as the light source 101, any light source which can emit low-coherent light such as an amplified spontaneous emission (ASE) light source, for example, may be used.

The light emitted from the light source 101 is guided to a fiber coupler 104 with a polarization holding function via a polarization maintaining (PM) fiber 102 and a polarization controller 103, and split into a measuring beam (hereinafter referred to as "measuring beam for a tomographic image" or "OCT measuring beam") and a reference beam corresponding to the measuring beam.

The polarization controller 103 is for adjusting the state of light emitted from the light source 101 to linear polarization. The branching ratio of the fiber coupler 104 is 90 (the reference beam) to 10 (the measuring beam).

The measuring beam is emitted as parallel light from a collimator 106 via a PM fiber 105. The emitted measuring beam reaches a dichroic mirror 111 via an X scanner 107 including a galvanometer mirror which scans the measuring beam in the horizontal direction at the fundus Er and a Y scanner 110 including a galvanometer mirror which scans the measuring beam in the vertical direction at lenses 108 and 109 and the fundus Er. The X and Y scanners 107 and 110 are controlled by a drive control unit 180 and can scan the measuring beam in a desired range of the fundus Er. The range in which the measuring beam is scanned on the fundus can be regarded as the acquisition range of the tomographic image, the acquisition position of the tomographic image, and the irradiation position of the measuring beam. The X and Y scanners 107 and 110 are examples of scanning unit for the PS-OCT and may be configured as a common XY scanner. The dichroic mirror 111 has a characteristic that reflects light with a wavelength of 800 nm to 900 nm and transmits the rest.

The measuring beam reflected by the dichroic mirror 111 passes through a $\lambda/4$ polarizing plate which is arranged with an angle of 45° tilted from P polarization to S polarization with an optical axis as a rotation axis via a lens 112 to shift the phase of the measuring beam by 90°, and is polarization controlled to a circularly polarized light. The $\lambda/4$ polarizing plate is an example of a polarization adjustment member for the measuring beam for adjusting the polarization state of the measuring beam. If the PS-SLO optical system described below is applied, the $\lambda/4$ polarizing plate 113 can be provided in a common optical path of a part of the PS-OCT optical system and a part of the PS-SLO optical system. This allows comparatively suppressing the dispersion of polarization state occurring on the image acquired by the PS-SLO optical system and the image acquired by the PS-OCT optical system. At this point, the scanning units for the PS-SLO and the PS-OCT are provided in positions conjugate to each other and can be provided in positions conjugate to the pupil of the subject's eye. The tilt of the $\lambda/4$ polarizing plate 113 is an example of state of the $\lambda/4$ polarizing plate 113 and an angle from a predetermined position with the optical axis of a polarization split face of a fiber coupler 123 incorporating a polarization beam splitter as a rotation axis, for example.

The $\lambda/4$ polarizing plate 113 can be attached and detached to and from the optical path. There may be a mechanical configuration for rotating the $\lambda/4$ polarizing plate 113 with the optical axis or an axis parallel to the optical axis as a rotation axis, for example. This makes it possible to realize a small apparatus which can simply switch between the SLO and PS-SLO optical systems. In addition, this makes it possible to realize a small apparatus which can simply switch between the OCT and PS-SLO optical systems.

The light incident on the subject's eye is polarization-controlled to a circularly polarized light by arranging the λ/4 polarizing plate tilted by 45°, however, the light may not be controlled to the circularly polarized light at the fundus Er depending on the characteristics of the subject's eye. For that reason, the tilt of the λ/4 polarizing plate can be fine-adjusted by controlling the drive control unit 180.

The measuring beam which is polarization-controlled to a circularly polarized light is focused on the retinal layer of the fundus Er via the anterior eye portion Ea of eye, which is the subject, by a focus lens 114 mounted on a stage 116. The measuring beam with which the fundus Er is irradiated is reflected and scattered by each retinal layer and returns to the fiber coupler 104 via the above optical path.

The reference beam branched by the fiber coupler 104 is emitted as a parallel light from a collimator 118 via a PM fiber 117. As is the case with the measuring beam, the emitted reference beam is polarization-controlled by a λ/4 polarizing plate 119 which is arranged at an angle of 22.5° tilted from P polarization to S polarization with an optical axis as a rotation axis. The λ/4 polarizing plate 119 is an example of a polarization adjustment member for the reference beam for adjusting the polarization state of the reference beam. The reference beam passes through a dispersion compensation glass 120, is reflected by a mirror 122 on a coherence gate stage 121, and returns to the fiber coupler 104. This means that the reference beam passes through the λ/4 polarizing plate 119 twice to cause linear polarization light to return to the fiber coupler 104.

The coherence gate stage 121 is controlled by a drive control unit 180 to cope with difference in eye's axial length. A coherence gate refers to a position corresponding to an optical path length of the reference beam in the optical path of the measuring beam. In the present exemplary embodiment, the optical path length of the reference beam is changed, however, a difference in an optical path length between the optical paths of the measurement and the reference beam has only to be changed.

The light returning to the fiber coupler 104 is combined with the reference beam to be interfering light (hereinafter referred also to as "combined light"). The interfering light is incident on the fiber coupler 123 incorporating the polarization beam splitter. The polarization beam splitter splits the interfering light into P and S polarized light which are different in a polarization direction with a split ratio of 50 : 50.

The P polarized light passes through a PM fiber 124 and a collimator 130, is separated by a grating 131, and received by a lens 132 and a line camera 133. Similarly, the S polarized light passes through a PM fiber 125 and a collimator 126, is separated by a grating 127, and received by a lens 128 and a line camera 129. The gratings 127 and 131 and the line cameras 129 and 133 are arranged in the direction according to each polarized light.

The light received by each of the line cameras 129 and 133 is output as an electric signal according to the strength of the light and received by a signal processing unit 190 being an example of a tomographic image generation unit.

The tilts of the λ/4 polarizing plates 113 and 119 can be automatically adjusted based on the tilt of polarization split face of the polarization beam splitter, but may be automatically adjusted with reference to a straight line connecting the center of optic disk of the fundus with the center of macula lutea of the fundus. It is desirable to have a tilt detection unit (not illustrated) for detecting the tilts of the λ/4 polarizing plates 113 and 119. The tilt detection unit can detect a present tilt and a predetermined tilt. Needless to say, the tilts of the λ/4 polarizing plates 113 and 119 may be detected based on the strength of the received light to adjust the tilts thereof so that a predetermined strength of light can be acquired. As described below, an object indicating the tilt is displayed on a graphic user interface (GUI) and a user may adjust the tilt using a mouse. The polarization beam splitter and the λ/4 polarizing plates 113 and 119 are adjusted with reference to the perpendicular direction as a polarization reference to acquire the similar effect.

<Configuration of PS-SLO 140>

The configuration of the PS-SLO 140 is described below.

A light source 141 is a semiconductor laser and emits light whose center wavelength is 780 nm, for example, in the present exemplary embodiment. The measuring beam emitted from the light source 141 (hereinafter referred to as "measuring beam for fundus image" or "SLO measuring beam") passes through a PM fiber 142, is polarization controlled to a linearly polarized light by a polarization controller 145, and emitted as parallel light from a collimator 143. The emitted light passes through a hole portion of a perforated mirror 144 and reaches a dichroic mirror 154 via a lens 155, an X scanner 146 including a galvanometer mirror which scans the measuring beam into the horizontal direction at the fundus Er, lenses 147 and 148, and a Y scanner 149 including a galvanometer mirror which scans the measuring beam into the vertical direction at the fundus Er. The X and Y scanners 146 and 149 are controlled by the drive control unit 180 and can scan a desired range on the fundus using the measuring beam. The X and Y scanners 146 and 149 are examples of the scanning unit for the PS-SLO and may be configured as a common XY scanner. The dichroic mirror 154 has a characteristic that reflects light with a wavelength of 760 nm to 800 nm and transmits the rest.

The measuring beam of liner polarization reflected by the dichroic mirror 154 passes through the same optical path as the measuring beam of the PS-OCT 100 and reaches the fundus Er.

The measuring beam with which the fundus Er is irradiated is reflected and scattered at the fundus Er, and reaches the perforated mirror 144 via the above-described optical path. The light reflected by the perforated mirror 144 passes through a lens 150, is split by a polarization beam splitter 151 into light different in polarization direction (P polarized light and S polarized light in the present exemplary embodiment), received by avalanche photo diodes (APD) 152 and 153, converted into a electric signal, and received by the signal processing unit 190, which an example of a fundus image generation unit.

The position of the perforated mirror 144 is conjugate to the position of a pupil of the subject's eye. The light passing through the periphery of the pupil among light in which the measuring beam with which the fundus Er is irradiated is reflected and scattered is reflected by the perforated mirror 144.

In the present exemplary embodiment, both of the PS-OCT and the PS-SLO use the PM fiber, however, the use of a single mode fiber (SMF) allows acquiring similar configuration and effect by controlling polarization using the polarization controller.

<Anterior eye imaging unit 160>

The anterior eye imaging unit 160 is described.

In the anterior eye imaging unit 160, the anterior eye portion Ea is irradiated by an illumination light source 115 composed of light emitting diodes (LED) 115-a and 115-b emitting illumination light with a wavelength of 1000 nm. The light reflected by the anterior eye portion Ea reaches a dichroic mirror 161 via the lens 114, the polarizing plate 113, the lens 112, and the dichroic mirrors 111 and 154. The dichroic mirror 161 has a characteristic that reflects light with a wavelength of 980 nm to 1100 nm and transmits the rest. The light reflected by the dichroic mirror 161 is received by an anterior eye camera 165 via lenses 162, 163, and 164. The light received by the anterior eye camera 165 is converted into an electric signal and received by the signal processing unit 190.

<Internal fixation lamp 170>

The internal fixation lamp 170 is described.

The internal fixation lamp 170 includes an internal fixation-lamp display unit 171 and a lens 172. A plurality of light emitting diodes (LD) arranged in a matrix form is used as the internal fixation-lamp display unit 171. A position where to turn on the light emitting diodes is changed according to a site desired to be imaged by controlling the drive control unit 180. The light emitted from the internal fixation-lamp display unit 171 is guided to the subject's eye via the lens 172. The light emitted from the internal fixation-lamp display unit 171 has a wavelength of 520 nm and a desired pattern is displayed by the drive control unit 180.

<Control unit 200>

The control unit 200 for controlling the entire apparatus is described below.

The control unit 200 includes the drive control unit 180, the signal processing unit 190, a display control unit 191, and a display unit 192.

The drive control unit 180 controls each unit as described above.

The signal processing unit 190 includes an image generation unit 193 and an image analysis unit 194. The signal processing unit 190 generates an image, analyzes the generated image, and generates visualization information of the analysis result based on the signals output from the line cameras 129 and 133, the APDs 152 and 153, and the anterior eye camera 165. The generation and analysis of an image are described below in detail.

The display control unit 191 displays on display unit 192 the images generated by the tomographic image generation unit and the fundus image generation unit and acquired by a fundus image acquisition unit (not illustrated) and a tomographic image acquisition unit (not illustrated) respectively on a display screen. The display unit 192 is a liquid crystal display, for example. The image data generated by the signal processing unit 190 may be transmitted by wire to the display control unit 191 or by wireless. In this case, the display control unit 191 can be regarded as an image processing apparatus. As an image system, the fundus image acquisition unit may include the SLO optical system, and the tomographic image acquisition unit may include the OCT optical system. In this specification, for the case of a subject except the subject's eye, a "fundus image (fundus luminance image)" may be paraphrased in a "planar image (planar luminance image) " and the "fundus image acquisition unit" may be paraphrased in a "planar image acquisition unit".

The display unit 192 displays display forms indicating various pieces of information described below under the control of the display control unit 191. The image data from the display control unit 191 may be transmitted by wire to the display unit 192 or by wireless. The display unit 192 is included in the control unit 200, however, the present invention is not limited thereto, and the display unit 192 may be provided separately from the control unit 200. Alternatively, a tablet may be provided, which is an example of a user portable apparatus into which the display control unit 191 and the display unit 192 are integrated. In this case, it is desirable to mount a touch panel function on the display unit to allow moving the display position of an image, expanding and reducing the image and changing the displayed image on the touch panel.

<Image processing>

The image generation by the image generation unit 193 included in the signal processing unit 190 is described below.

The image generation unit 193 subjects the interference signals output from the line cameras 129 and 133 to a re-configuration processing used in a general spectral domain OCT (SD-OCT) to generate tomographic images corresponding to a first and a second polarized light which are two tomographic images based on each polarization component.

The image generation unit 193 reduces a fixed pattern noise from the interference signal. The fixed pattern noise is reduced in such a manner that a plurality of the detected A scan signals is averaged to extract the fixed pattern noise, and the extracted fixed pattern noise is subtracted from the input interference signal.

The image generation unit 193 converts the interference signal from wavelength to the number of waves and performs Fourier-transform thereof to generate a tomographic signal indicating a polarization state.

The interference signal of two polarization components is subjected to the above processing to generate two tomographic images.

The image generation unit 193 aligns the signals output from the APDs 152 and 153 in synchronization with the drive of the X and Y scanners 146 and 149 to generate fundus images corresponding to the first and the second polarized light which are two fundus images based on each polarization component.

<Generation of tomographic luminance image or fundus luminance image>

The image generation unit 193 generates the tomographic luminance image from the two tomographic signals.

The tomographic luminance image is basically the same as the tomographic image in the conventional OCT. The pixel value r is calculated from the tomographic signals $A_H$ and $A_V$ acquired from the line sensors 129 and 133 by an equation 1:

$$r = \sqrt{A_H^2 + A_V^2} \qquad \text{(Equation 1)}$$

Similarly, the image generation unit 193 generates the fundus luminance image from the two fundus luminance images.

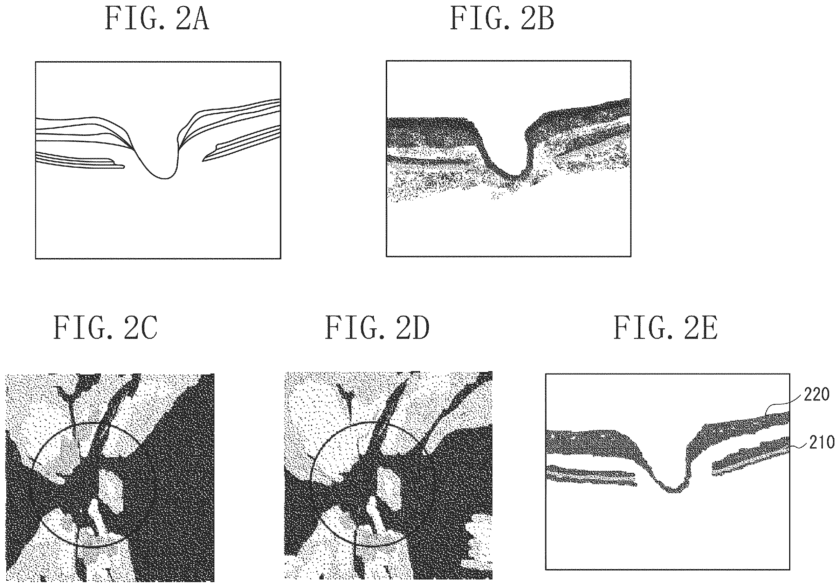

Fig. 2A illustrates an example of a luminance image of an optic disk portion.

The display control unit 191 may cause the display unit 192 to display the tomographic luminance image acquired by a conventional OCT method on the display unit 192 if the λ/4 polarizing plate 113 is removed from the optical path or the fundus luminance image acquired by a conventional SLO method on the display unit 192.

<Generation of retardation image>

The image generation unit 193 generates a retardation image from a tomographic image of polarization components orthogonal to each other.

A value δ of each pixel of the retardation image is a value indicating a ratio of influence of vertical and horizontal polarization components on the subject's eye in a position of each pixel forming the tomographic image. The value δ is calculated from the tomographic signals $A_H$ and $A_V$ by an equation 2:

$$\delta = \arctan\left[\frac{A_V}{A_H}\right] \qquad \text{(Equation 2)}$$

Fig. 2B illustrates an example of a retardation image of thus generated optic disk portion, and the retardation image can be obtained by the equation 2 for each B scan image. As described above, the retardation image refers to the tomographic image indicating a difference in influence of two polarizations on the subject's eye. In Fig. 2B, the value indicating the ratio is displayed in color as the tomographic image. Places where shading is thick are small in value indicating the ratio. Places where shading is thin are large in value indicating the ratio. For this reason, the generation of the retardation image allows a birefringent layer to be recognized. The details are as discussed in "E. Gotzinger et al., Opt. Express 13, 10217, 2005".

Similarly, the signal processing unit 190 can also generate the retardation image in the planar direction of the fundus based on the output from the APDs 152 and 153.

<Generation of retardation map>

The image generation unit 193 generates a retardation map from the retardation image acquired from a plurality of B scan images.

The image generation unit 193 detects a retinal pigment epithelium (hereinafter referred to as "RPE") in each B scan image. The RPE has the property of dissolving polarization, so that the distribution of retardation is examined along a depth direction without the range from an internal limiting membrane (hereinafter referred to as "ILM") to the RPE by each A scan and the maximum value of the retardation is taken as the representative value of the retardation in the A scan.

The image generation unit 193 performs above processing on all retardation images to generate a retardation map.

Fig. 2C illustrates an example of the retardation map of the optic disk portion. Places where shading is thick are small in value indicating the ratio. Places where shading is thin are large in value indicating the ratio. In the optic disk portion, a layer with birefringence is a retinal nerve fiber layer (hereinafter, referred also to as "RNFL"). The retardation map is an image indicating a difference in influence which two polarizations receive according to the birefringence of the RNFL and the thickness of the RNFL. For this reason, the value indicating the ratio is increased at a place where the RNFL is thick and decreased at a place where the RNFL is thin. Therefore, with the retardation map, the thickness of the RNFL all over the fundus can be recognized, and the retardation map can be used for the diagnosis of glaucoma.

<Generation of birefringence map>

The image generation unit 193 linearly approximates the value of the retardation $\delta$ in the range from the ILM to the RNFL in each A scan image of the previously generated retardation image and determines the tilt of the retardation $\delta$ as birefringence in a position on the retina of the A scan image. In other words, the retardation is the product of distance and birefringence in the RNFL, so that the values of depth and retardation in each A scan image are plotted to acquire a linear relationship. Therefore, if the tilt is acquired by linearly approximating the plot using the least squares method, the tilt is a value of the birefringence of the RNFL in the A scan image. All the retardation images are subjected to the processing to generate the map indicating birefringence.

Fig. 2D illustrates an example of the birefringence map of the optic disk portion. Because birefringence values are directly mapped, the birefringence map can describe a fiber structure as a change in birefringence if the fiber structure is changed even if the thickness of the RNFL is not changed.

[Generation of DOPU image]

The image generation unit 193 calculates a Stokes vector S for each pixel from the acquired tomographic signals $A_H$ and $A_V$ and a phase difference $\Delta\phi$ between the tomographic signals using an equation 3.

$$S = \begin{pmatrix} I \\ Q \\ U \\ V \end{pmatrix} = \begin{pmatrix} A_H^2 + A_V^2 \\ A_H^2 - A_V^2 \\ 2A_H A_V \cos\Delta\phi \\ 2A_H A_V \sin\Delta\phi \end{pmatrix} \qquad \text{(Equation 3)}$$

Where, the phase difference $\Delta\phi$ is calculated from phases $\phi_H$ and $\phi_V$ of each signal acquired when two tomographic signals are calculated as $\Delta\phi = \phi_V - \phi_H$.

The image generation unit 193 sets a window with a size of about 70 $\mu$m in the main scanning direction of the measuring beam and about 18 $\mu$m in the depth direction thereof in each B scan image, averages each element of a stroke vector calculated for each pixel by a number C in each window and calculates the degree of polarization uniformity (DOPU) of polarization in the window using an equation 4.

$$DOPU = \sqrt{Q_m^2 + U_m^2 + V_m^2} \qquad \text{(Equation 4)}$$

Where, $Q_m$, $U_m$, and $V_m$ are values in which the elements Q, U, and V of the stroke vector in each window are averaged. All the windows in the B scan image are subjected to the processing to generate the DOPU image of the optic disk portion illustrated in Fig. 2E. As described above, the DOPU image is the tomographic image indicating the degree of uniformity of two polarizations.

The DOPU is a value indicating the degree of uniformity of polarization and is the value near to 1 in a place where polarization is maintained, but is the value smaller than 1 in a place where polarization is dissolved and not maintained. In a structure of a retina, the RPE has the property of dissolving polarization, so that the value is smaller than that in other areas in a portion corresponding to the RPE in the DOPU image. In the figure, a place 210 where shading is thin represents the RPE and a place 220 where shading is thick represents the area of the retinal layer where change is maintained. The DOPU image images a layer resolving polarization such as the RPE, so that the DOPU image can more surely image the RPE than change in luminance even if the RPE is deformed due to disease.

Similarly, the signal processing unit 190 can also generate the DOPU image in the planar direction of the fundus based on the output from the APDs 152 and 153.

In the present specification, the tomographic image, the retardation image, and the DOPU image corresponding to the first and the second polarization described above are also referred to as a tomographic image representing the polarization state. Also in the present specification, the retardation map and the birefringence map described above are also referred to as a fundus image representing the polarization state.

<Processing operation>

The processing operation of the image processing apparatus is described below.

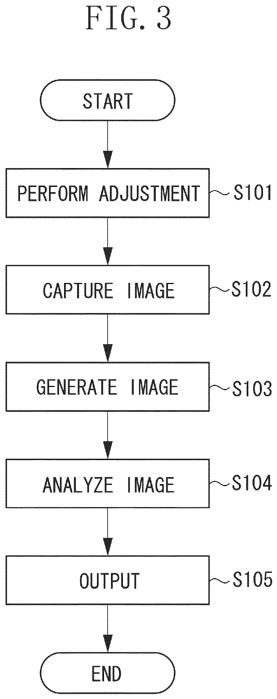

Fig. 3 is a flow chart illustrating the processing operation of the image processing apparatus.

<Adjustment>

In step S101, the subject's eye is placed on the apparatus and the apparatus is aligned with the subject's eye. For the description of alignment, only specific processing of the present exemplary embodiment is described. The descriptions of alignment in the XYZ directions such as working distance, and the adjustment of focus and coherence gate are omitted because they are general.

<Adjustment of PS-OCT imaging position>

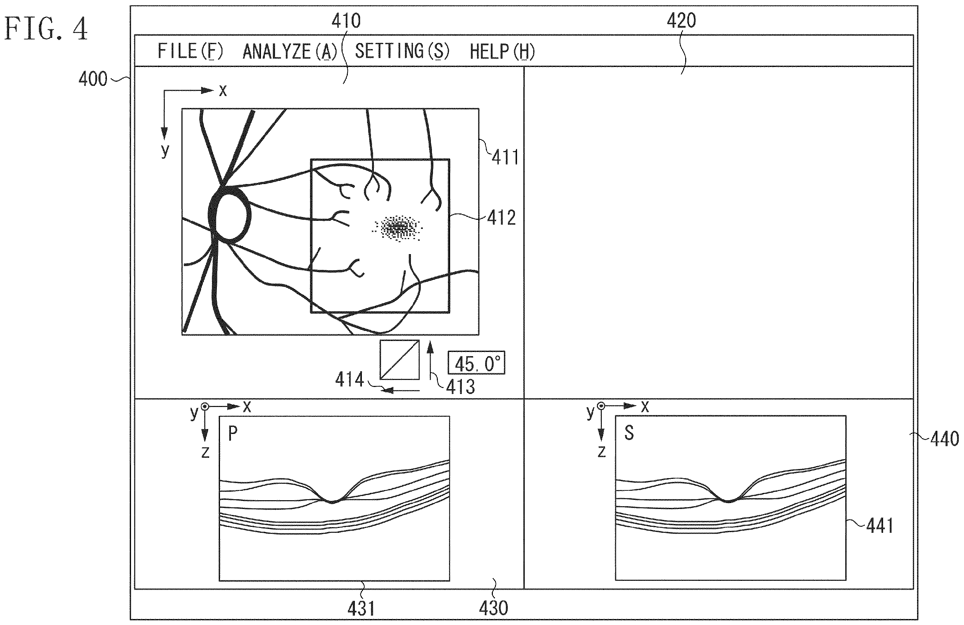

Fig. 4 illustrates a window 400 displayed in the display unit 192 during adjustment. A fundus image 411 imaged by the PS-SLO 140 and generated by the signal processing unit 190 is displayed in a display area 410 which is an example of a first display area and a frame 412 indicating the imaging range of the PS-OCT 100 is superimposed and displayed on the fundus image 411.

The imaging range is set under the control of the drive control unit 180 in such a manner that the imaging range is specified by a cursor displayed on the window 400 and by performing click and drag operation using an instruction apparatus (not illustrated) such as a mouse. In other words, the frame 412 is specified by the cursor and drag operation is performed to move the frame 412. With this operation, the drive control unit 180 controls the drive angle of the scanner to set the imaging range. The mouse according to the present exemplary embodiment is provided with a sensor which detects a movement signal when a mouse main body is two-dimensionally moved by a user's hand, for example, a left and a right mouse buttons for detecting the pressing by the user's hand, and a wheel mechanism which can be rotated back and forth and left and right between the left and the right mouse button. A display unit of the instruction apparatus may be provided with a touch panel function to specify an acquisition position on the touch panel.

<Adjustment of λ/4 polarizing plate>

The adjustment of the λ/4 polarizing plate 113 is described below.

In Fig. 4, instruction portions 413 and 414 are displays to be used for adjusting the angle of the λ/4 polarizing plate 113. The operator issues instructions using the instruction apparatus to adjust the angle of the λ/4 polarizing plate 113 under the control of the drive control unit 180. The instruction portion 413 is a display for instructing a counterclockwise adjustment. The instruction portion 414 is a display for instructing a clockwise adjustment. A numeric value displayed at the side of the instruction portions 413 and 414 indicates the present angle of the λ/4 polarizing plate 113. The display control unit 191 may arrange the instruction portion for adjusting the angle of the λ/4 polarizing plate 119 alongside of the instruction portion 413 and display the instruction portion on the display unit 192, or may display the instruction portion instead of the instruction portion 413on the display unit 192.

The operator issues an instruction with a cursor using the mouse so that the luminance of the tomographic image of each polarization displayed in a display area 430, which is an example of a third display area, and a display area 440, which is an example of a fourth display area becomes equal to each other. It may be possible to display a peak luminance value along with the tomographic images 431 and 441 of respective polarizations or waveforms themselves of respective interference signals to perform adjustment while viewing them. The tomographic images 431 and 441 of each polarization are examples of the tomographic images corresponding to the first and the second polarization. It is desirable that a display form indicating the type of each image, in other words, a character "P" representing P polarization or a character "S" representing S polarization, for example, is superimposed on the tomographic images 431 and 441 of each polarization (or tomographic images 531 and 541, described below) and displayed. This can prevent the user from erroneously recognizing an image. It is needless to say that the character may be displayed above or at the side of the image without being superimposed on the images or displayed correspondingly with the image.

In this stage, there is no need for displaying anything in a display area 420 which is an example of a second display area. In the case of automatic adjustment, a display form indicating the present adjustment state, that is, a message "λ/4 polarizing plate is being adjusted", for example, may be displayed. The window 400 may display a display form indicating patient information such as the left and right eyes of the subject's eyes or indicating imaging information such as an imaging mode. It is desirable to repeat attachment and detachment of the λ/4 polarizing plate 113 to and from the optical path to alternately acquire the fundus luminance image and the tomographic image indicating the polarization state. With this operation, in an ophthalmologic apparatus as small as possible, the display control unit 191 can display the fundus luminance image in the display area 410and display the tomographic image indicating the polarization state in the display area 420, for example.

It is desirable to perform adjustment in the following order: alignment adjustment using the anterior-eye portion image or a luminescent spot in a cornea; focus adjustment using the fundus image indicating the polarization state; coherence gate adjustment using the tomographic image indicating the polarization state; and adjustment of the λ/4 polarizing plate. A position where the tomographic image indicating the polarization state is acquired is desirably determined before the coherence gate adjustment using the tomographic image indicating the polarization state, however, the position may be determined at an initial setting to acquire the center area of the tomographic image indicating the polarization state. Thereby, the ophthalmologic apparatus can be easily adjusted so that the tomographic image indicating the polarization state targeting a finer and narrower area than the fundus image indicating the polarization state can be accurately and easily obtained. At this point, the λ/4 polarizing plate may be automatically adjusted upon the completion of the coherence gate adjustment or upon the input of a signal for acquiring the image indicating the polarization state. Needless to say, the λ/4 polarizing plate may be previously adjusted on an initial setting screen in starting the ophthalmologic apparatus to eliminate the need for adjustment for each imaging.

If the λ/4 polarizing plate can be attached and detached to and from the optical path, it is desirable to perform adjustment in the following order: alignment adjustment using the anterior-eye portion image or a luminescent spot in a cornea; focus adjustment using the SLO fundus image; coherence gate adjustment using the OCT tomographic image; insertion of the λ/4 polarizing plate into the optical path; and adjustment of the λ/4 polarizing plate. Adjustment can thus be performed before acquiring the image indicating the polarization state, using the normal SLO fundus image and the OCT tomographic image which the user is accustomed to and can perform intuitively. The coherence gate may also be adjusted using the tomographic image indicating the polarization state of the PS-OCT after inserting the λ/4 polarizing plate after performing focus adjustment. At this point, the λ/4 polarizing plate may be automatically inserted into the optical path in response to completion of adjustment of the coherence gate or in response to completion of adjustment of focus, or the λ/4 polarizing plate may be automatically inserted into the optical path in response to the input of a signal for acquiring the image indicating the polarization state.

The focus may be finely adjusted using the OCT tomographic image after coarsely adjusting the focus using the SLO fundus image.

All of such adjustments may be automatically performed in the above order or by the user adjusting the cursor to a slider corresponding to each adjustment displayed on the display unit and performing dragging operation. If the λ/4 polarizing plate is attached or detached, an icon for instructing the λ/4 polarizing plate to be attached or detached to and from the optical path may be displayed on the display unit.

<Imaging> to <Image formation>

In step S102 and step S103, each of the light sources 101 and 141 emits the measuring beam. The line cameras 129 and 133 and the APDs 152 and 153 receive the return beam from the fundus Er, and the image generation unit 193 generates each image as described above. In the present exemplary embodiment, the X and Y scanners 107 and 110 are controlled to generate N B-scan images each composed of M A-scan images. The M and N can be set based on time required for imaging and the size of an area required for diagnosis. Values of the M and N have only to be set to approximately 1024 and 250 respectively for an area of about 8 mm x 6 mm with macula lutea as a center, for example.

<Analysis>

In step S104, the image analysis unit 194, which is an example of the extraction unit, calculates the DOPU using the equation 4 to extract the area where the polarization is canceled in the retinal layer (hereinafter referred also to as "depolarized area"). The depolarized area refers to an area which is relatively large in a difference in influence of two polarizations on the subject's eye. The depolarized area is also called a predetermined area. If the RPE is distorted by atrophy, the distorted region is not detected as the RPE in the DOPU, which brings the advantage that the atrophic region of the RPE can be detected. The distorted region of the RPE indicates a region where the RPE is discontinuous or a defective region (an abnormal region in the RPE). A display form indicating the type of a discontinuous region can be displayed on the display unit based on a state (such as continuity) at a discontinuous region in the extracted depolarized area. This can provide the user with information effective for diagnosis depending on the state of an atrophic region in the depolarized area.

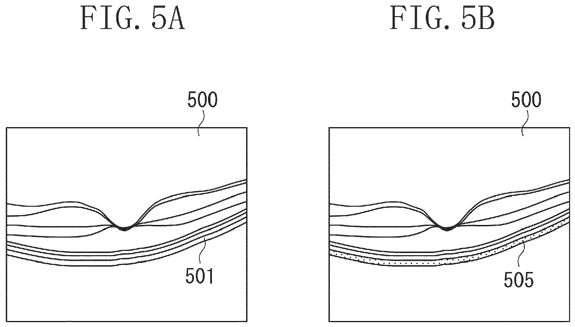

Fig. 5A illustrates a tomographic image 500 acquired by imaging the macula lutea portion of a normal subject and a RPE 501. Fig. 5B is an example in which a depolarized area 505 acquired by calculating the DOPU is superimposed on the tomographic image. As illustrated in Figs. 5A and 5B, the RPE 501 is in the depolarized area in the retinal layer, so that the depolarized area 505 is superimposed on the RPE 501.

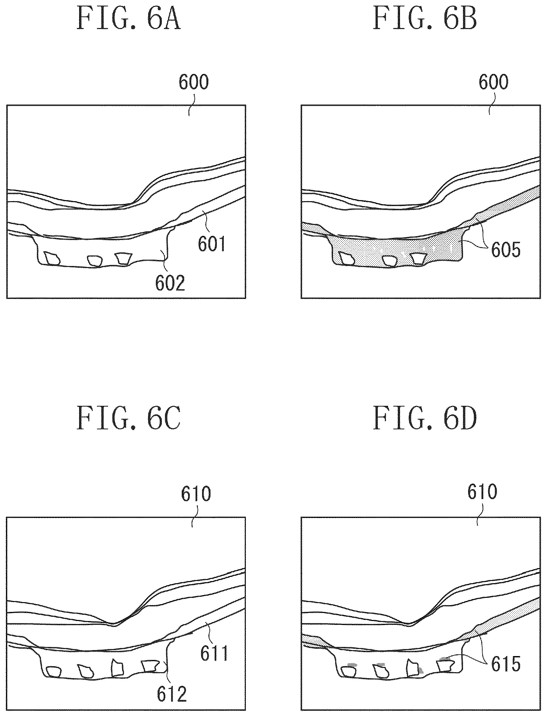

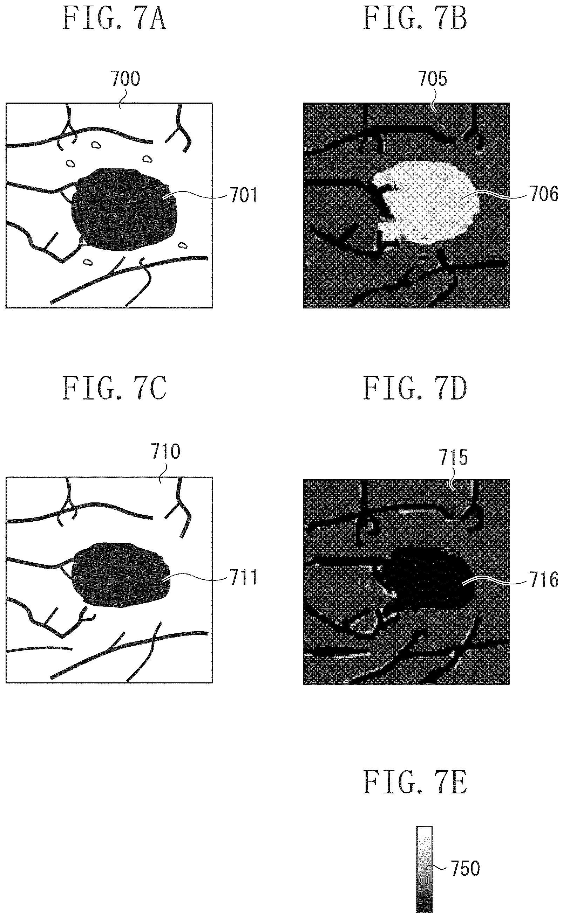

Figs. 6A to 6D illustrate examples in which a depolarized area is superimposed on a tomographic image in a case where a lesion exists in an eye. Fig. 6A illustrates a tomographic image 600, the RPE 601, and a choroid coat 602 in a case of Stargardt disease. Fig. 6B is an example in which a depolarized area 605 acquired by calculating the DOPU is superimposed on the tomographic image. Fig. 6C illustrates a tomographic image 610, the RPE 611, and a choroid coat 612 in a case of age-related macular degeneration and geographic atrophy. Fig. 6D is an example in which a depolarized area 615 acquired by calculating the DOPU is superimposed on the tomographic image. As illustrated in Figs. 6A and 6C, it can be difficult to distinguish a difference only by comparing the tomographic image 600 with the tomographic image 610, however, as illustrated in Figs. 6B and 6D, the depolarized area is detected to allow distinguishing a difference between the tomographic images 600 and 610. Figs. 7A and 7C illustrate fundus autofluorescence images. Figs. 7B and 7D illustrate examples of maps representing the thickness of the depolarized area. The map of the thickness of the depolarized area represents a three dimensional data as a two dimensional map by measuring the thickness in the depth direction of the depolarized area 605 illustrated in Fig. 6B in a plurality of the imaged tomographic images. Fig. 7A illustrates the fundus autofluorescence image of Stargardt disease. Fig. 7B illustrates an example of map of the thickness of the depolarized area thereof. Fig. 7C illustrates the fundus autofluorescence image of age-related macular degeneration. Fig. 7D illustrates an example of map of the thickness of the depolarized area thereof. Areas 701 and 711 in Figs. 7A and 7C represent areas where the RPE disappears or thins, respectively. The map of the thickness of the depolarized area illustrated in Figs. 7B and 7D represents that color is light in a portion where the depolarized area is thick in thickness and deep in a portion where the area is thin in thickness, as indicated by a color bar 750 in Fig. 7E. As illustrated in Figs. 7A and 7C, it can be difficult to distinguish a difference only by comparing the fundus autofluorescence images with each other, however, as illustrated in Figs. 7B and 7D, the depolarized area is represented by the thickness map to allow distinguishing a difference between them.

The image analysis unit 194, which is an example of a determination unit, determines a type of a discontinuous region based on a discontinuous state (such as continuity) of the above depolarized area. In the map of the thickness of the depolarized area, for example, if the width of distribution of an area where the thickness of the depolarized area exceeds a certain value (presumed to be 50 $\mu$m or more) is larger than a threshold value (presumed to be 10% or more), Stargardt disease may be caused. If the width of distribution of an area where the thickness of the depolarized area is equal to or smaller than a certain value (presumed to be 5 $\mu$m or less) is smaller than the threshold value, or if the width of distribution of an area where the depolarized area does not exist is larger than the threshold value (presumed to be 10% or more), geographic atrophy of age-related macular degeneration may be caused. Thus, according to whether the width of distribution in a discontinuous region in the direction crossing a depth direction is greater or smaller than the threshold value, the type of a lesion in the discontinuous region can be determined as the type of the discontinuous region. Alternatively, the image analysis unit 194 may include a discriminator such as a support vector machine and an AdaBoost, and cause the discriminator to determine the type of disease using characteristics of the tomographic image and the depolarized area.

It can be difficult to distinguish a difference between different diseases such as Stargardt disease (retinal degeneration due to genetic mutation) and geographic atrophy of age-related macular degeneration on a normal OCT image (a tomographic luminance image).

On the other hand, a difference between the diseases can be distinguished in the polarization-sensitive tomographic image. Disease is automatically determined using the polarization-sensitive tomographic image.

More specifically, disease is automatically discriminated based on the state of the discontinuous region in the predetermined area (depolarized area) of a polarization-sensitive tomographic image such as the RPE layer to present the type of disease (the name of disease) to the user. Thus, the polarization-sensitive tomographic image is used to allow the user to early find a very small change in the RPE layer caused by disease to recognize the type of the disease early.

The state of the discontinuous region refers to the thickness, width, size, area of the RPE layer and the connectivity between adjacent pixels, for example, and any parameters indicating discontinuity may be used. The state of the discontinuous region may be the volume of the discontinuous region on the RPE layer in the three-dimensional polarization-sensitive tomographic image. The comparison of a parameter indicating discontinuity with the threshold value allows determining the type of disease according to comparison results. A reference for comparing sizes such as thickness and width of a discontinuous region on the RPE layer may be extracted from the tomographic luminance image . An area where a luminance value is large but the polarization is not randomized (Bruch membrane, for example) is extracted from the tomographic luminance image and the extracted area may be taken as the reference.

<Output>

An output processing for generated images and analysis results performed in step S105 is described below. The output processing in the present exemplary embodiment effectively displays the characteristics of the depolarized area acquired in step S104.

When the image generation unit 193 and the image analysis unit 194 in the signal processing unit 190 finish generating and analyzing images, the display control unit 191 generates output information based on the results, and outputs and displays the output information on the display unit 192.

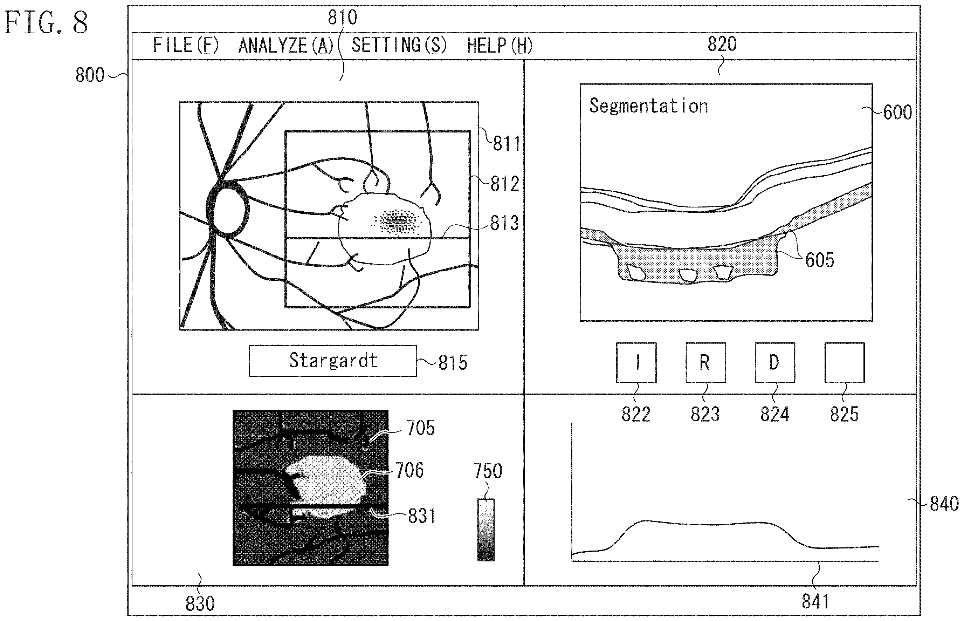

Fig. 8 is a display example on the display unit 192 according to the present exemplary embodiment. In Fig. 8, a window 800 is displayed on the display unit 192 and includes display areas 810, 820, 830, and 840.

A fundus image 811 is displayed in a display area 810, which is a first display area. A rectangular frame 812 indicating an imaging position of a tomographic image and a line 813 indicating a position of a tomographic image 600 are superimposed on the fundus image 811. The fundus image 811 was imaged by the PS-SLO. A display area 815 for displaying the type of disease estimated using characteristics of the depolarized area acquired in step S104 exists in the vicinity of the fundus image 811. In the present exemplary embodiment, the case of Stargardt disease is illustrated as an example. In the example in Fig. 8, only the name of the estimated disease is displayed, however, other than that, approximately three types of lesions high in probability may be displayed in the descending order of the probability. In that case, it is desirable to display not only the name of a lesion, but also the probability thereof. If such a display is performed, results of estimating the name of a lesion using the discriminator are displayed.

The tomographic image 600 is displayed in a display area 820, which is a second display area. Buttons 822 to 825, which are examples of a selection portion for selecting the type of a displayed tomographic image are displayed in the display area 820. The type of a tomographic image may be selected from the menu instead of the buttons 822 to 825. Fig. 8 illustrates an example state where the button 825 is selected and segmentation results are superimposed on a tomographic luminance image to highlight a depolarized area 605. The other buttons 822 to 824 and the display thereof are described below. A tomographic luminance image is displayed in the display area 820 by the user selecting the button 822. A retardation image is displayed in the display area 820 by the user selecting the button 823. A DOPU image is displayed in the display area 820 by the user selecting the button 824.

A thickness map 705 of the depolarized area is displayed in the display area 830, which is an example of a third display area. The thickness map 705 of the depolarized area is described in Fig. 7. A line 831 indicating the position of the tomographic image 600 is superimposed on the thickness map 705 of the depolarized area. The line 831 also corresponds to the line 813. A graph 841 indicating the thickness of the depolarized area is displayed in the display area 840, which is an example of a fourth display area. Thereby, the user can confirm a change in structure associated with the structure of the retinal layer in the PS-OCT to allow effectively diagnosing the subject's eye.

The display control unit 191 may display the retardation map or the birefringence map on any of the display areas of the display unit 192 instead of the above mentioned various types of images. The display control unit 191 may superimpose the retardation map or the birefringence map on the fundus luminance image 811 and display them. At that point, it is desirable that the retardation map or the birefringence map is superimposed on the area indicated by the frame 812. As described above, according to the present exemplary embodiment, information about the polarization components acquired from the polarization-sensitive OCT image is used to allow displaying information about a depolarization material and providing information in which the probability of a lesion is estimated.

The position of the display area for displaying the images is not limited to that in the present exemplary embodiment, and the fundus image may be displayed in the display area on the left side of the display screen, for example. The number of images to be displayed is not limited to that in the present exemplary embodiment, and both of the fundus image and the tomographic image may be displayed side by side on the display screen at the time of adjustment, for example, and a display system is changed after the imaging and a plurality of tomographic images indicating polarization states different from each other as well as the fundus image can be displayed side by side on the display screen. An arrangement order of the buttons 822 to 825 and the position thereof are not limited to those in the present exemplary embodiment.

<Determination of type classified in specific disease>

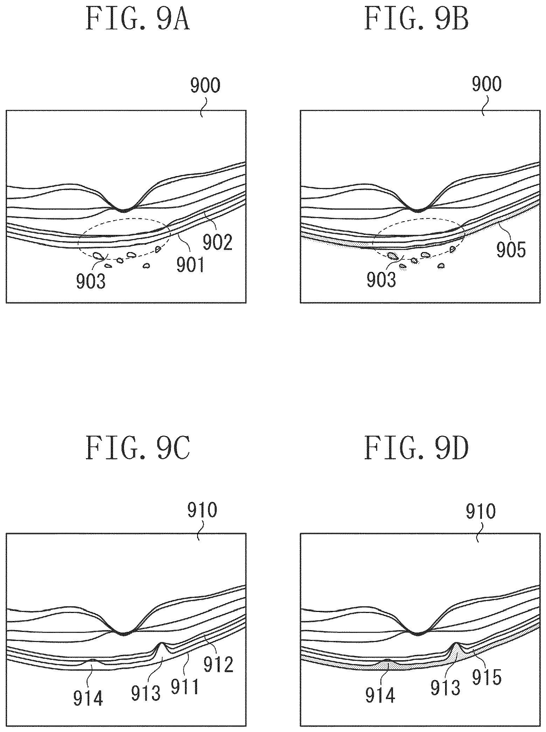

An example is described below with reference to Fig. 9, where the type is determined in which a specific disease such as Stargardt disease, for example, is further finely classified for a discontinuous region. The present exemplary embodiment is also an example where the type of a discontinuous region is determined.

Fig. 9A illustrates a tomographic image 900, an RPE 901, and an IS/OS 902 in a case where a photoreceptor cell inner segment/outer segment (hereinafter referred to as IS/OS) disappears and atrophy or defect occurs in the RPE due to the Stargardt disease. Fig. 9B is an example in which a depolarized area 905 acquired by calculating the DOPU is superimposed on the tomographic image. A circular area 903 indicated by a broken line is an example of a display form representing the discontinuous region of the depolarized area and illustrates an area where the IS/OS disappears. As illustrated in Fig. 9B, in the area where the IS/OS disappears, it can be grasped that the depolarized area 905 does not exists in an area which seems to be the RPE and the RPE is atrophied. In other words, the atrophy of the RPE which is hard to determine from the tomographic image 900, which is the luminance image, can be easily grasped by acquiring the depolarized area.

Fig. 9C illustrates a tomographic image 910, an RPE 911, and an IS/OS 912 in a case where the RPE infiltrates into an entoretina layer due to Stargardt disease. Fig. 9D is an example in which a depolarized area 915 acquired by calculating the DOPU is superimposed on the tomographic image. Regions 913 and 914 are portions that infiltrate into an entoretina layer in the depolarized area because lesion exists in the RPE. Fig. 9D illustrates the region 913 reaching the IS/OS in the depolarized area, and the region 914 remaining under the IS/OS. The IS/OS is a photoreceptor cell junction. The existence of a lesioned part in the junction may lead to visual deficit.

The image analysis unit 194, which is an example of a determination unit determines the type of a discontinuous region based on a discontinuous state of the above depolarized area. For example, the image analysis unit 194 detects the border of a retinal layer and views a positional relationship between the position of the border and the depolarized area or the continuity of the depolarized area to estimate the type of a lesion. As the border of the retinal layer, the border of the RPE and the IS/OS is detected from the tomographic images 900 and 910. The border is detected in such a manner that a median filter as a kind of smoothing unit and a Sobel filter as a kind of edge detection unit are applied to the tomographic image to be processed to generate each image (hereinafter, referred to as "median image" and "Sobel image"). Then, a profile is generated from the generated median image and Sobel image for each A scan. Profiles of a luminance value and a gradient are generated from the median image and the Sobel image, respectively. Their borders are detected with reference to the profile of the median image corresponding to the before and after of the detected peak and between the peaks. The detected border may be used as it is and an n-th degree curve is applied based on the detected border to estimate a reference line. In the present exemplary embodiment, a secondary curve is applied to the border of the RPE detected by the image analysis unit 194 and taken as the reference line. A robust estimation such as M estimation or Least Median of Squares (LMedS) is used for the estimation of a secondary curve. A positional relationship between the positions of these borders and the depolarized area is utilized thereto.

The image analysis unit 194 performs estimation using characteristics whether the depolarized area is continuous with respect to the RPE border or the reference line or where the depolarized area exists with respect to the RPE border or the reference line in the discontinuous range. If the IS/OS disappears and atrophy or defect occurs in the RPE due to the Stargardt disease, there are the discontinuity of the depolarized area in an RPE atrophy range and the depolarized area under the RPE border (in the depth direction) . If the RPE infiltrates into the entoretina layer due to Stargardt disease, there is a region where the depolarized area is partly thick and the area reaches the IS/OS border or the vicinity of the IS/OS border. If the depolarization material diffuses into the choroid coat due to the above Stargardt disease, which means that there is an area where the thickness of the depolarized area exceeds a certain value (presumed to be 50 $\mu$m) under the RPE border (in the depth direction) in a wide range. Therefore, the image analysis unit 194 can estimate a difference in the type using the characteristics.

[Other exemplary embodiments]

The present invention is realized by executing the following processing. Software (programs) realizing the functions of the above exemplary embodiments is supplied to a system or an apparatus via a network or various types of storage media and a computer (or a central processing unit (CPU) or a micro processing unit (MPU)) of the system or the apparatus reads the programs and performs processing.

According to the present invention, a display form indicating the type of a discontinuous region can be displayed on the display unit based on the state (continuity) of the discontinuous region in the area where the polarization extracted from the polarization-sensitive tomographic image of the subject is randomized. Thereby, information effective for diagnosis can be provided for the user depending on the state of an atrophic region in the area where the polarization is randomized.

Embodiments of the present invention can also be realized by a computer of a system or apparatus that reads out and executes computer executable instructions recorded on a storage medium (e.g., non-transitory computer-readable storage medium) to perform the functions of one or more of the above-described embodiment (s) of the present invention, and by a method performed by the computer of the system or apparatus by, for example, reading out and executing the computer executable instructions from the storage medium to perform the functions of one or more of the above-described embodiment (s) . The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

**Claims**

1.  An image processing apparatus comprising:

    a tomographic image acquisition means (100) configured to acquire a polarization-sensitive tomographic image (600) of a subject (Er);
    an extraction means configured to extract a predetermined area from the acquired polarization-sensitive tomographic image;
    a determination means (194) configured to determine a type of disease based on a state at a discontinuous region (605) in the extracted predetermined area; and
    a display control means configured to cause a display means (192) to display the type of disease determined by the determination means (194).

2.  The image processing apparatus according to claim 1, wherein the determination means is configured to determine the type of disease based on a connectivity between adjacent pixels of the discontinuous region.

3.  The image processing apparatus according to claim 2, wherein the determination means is configured to determine a type of lesion at the discontinuous region as the type of disease according to whether or not a width of distribution of the discontinuous region in the direction crossing the depth direction as seen on the tomographic image is larger than a threshold.

4.  The image processing apparatus according to any one of claims 1 to 3, wherein the display control means is configured to cause the display means to display the type of disease based on a parameter indicating the discontinuity of the discontinuous region.

5.  The image processing apparatus according to any one of claims 1 to 4, wherein the display control means is configured to cause the display means to display the type of disease based on at least one of the thickness and the width of the discontinuous region as seen on the tomographic image.

6.  The image processing apparatus according to any one of claims 1 to 5,
    wherein the predetermined area is a depolarized area where the polarization is randomized, and
    wherein the display control means is configured to cause the display means to display the depolarized area superimposed on the tomographic luminance image of the subject and to cause the display means to display the display form indicating the discontinuous region with the display form superimposed on the tomographic luminance image.

7.  The image processing apparatus according to any one of claims 1 to 6, wherein the polarization-sensitive tomographic image is a tomographic image generated based on polarized lights different from each other and acquired by dividing light in which a return beam returned from the subject irradiated with a measuring beam is combined with a reference beam corresponding to the measuring beam.

8. An image processing method (S104) comprising:

acquiring a polarization-sensitive tomographic image of a subject;
extracting a predetermined area from the acquired polarization-sensitive tomographic image;
determining a type of disease based on a state at a discontinuous region (605) in the extracted predetermined area; and
causing a display means to display (S105) the type of disease determined in the determining step.

9. The image processing method according to claim 8, wherein, in the determining step, the type of disease is determined based on a connectivity between adjacent pixels of the discontinuous region.

10. The image processing method according to claim 9, comprising determining a type of lesion at the discontinuous region as the type of disease according to a size with respect to the threshold value of width of distribution of the discontinuous region in a direction crossing a depth direction as seen on the tomographic image.

11. The image processing method according to any one of claims 8 to 10, comprising causing the display means to display the type of disease based on a parameter indicating the discontinuity of the discontinuous region.

12. The image processing method according to any one of claims 8 to 11, comprising causing the display means to display the type of disease based on at least one of the thickness and the width the discontinuous region as seen on the tomographic image.

13. The image processing method according to any one of claims 8 to 12,
wherein the predetermined area is a depolarized area where the polarization is randomized, and
the method comprises causing the display means to display the depolarized area superimposed on the tomographic luminance image of the subject, and further causing the display means to display the display form indicating the discontinuous region superimposed on the tomographic luminance image.

14. The image processing method according to any one of claims 8 to 13, wherein the polarization-sensitive tomographic image is a tomographic image generated based on polarized lights different from each other and acquired by dividing light in which a beam returned from the subject irradiated with a measuring beam is combined with a reference beam corresponding to the measuring beam.

15. A computer-executable program for causing a computer to execute each step of the image processing method according to any one of claims 8 to 14.

**Patentansprüche**

1. Bildverarbeitungsvorrichtung, umfassend:

eine Tomographiebild-Erfassungseinrichtung (100), die konfiguriert ist, ein mit polarisationsempfindlicher Tomographie gewonnenes Bild, kurz: polarisationsempfindliches Tomographiebild, (600) eines Subjekts (Er) zu erfassen;
eine Extraktionseinrichtung, die konfiguriert ist, ein vorbestimmtes Gebiet aus dem erfassten polarisationsempfindlichen Tomographiebild zu extrahieren;
eine Bestimmungseinrichtung (194), die konfiguriert ist, einen Krankheitstyp basierend auf einem Zustand an einer diskontinuierlichen Region (605) im extrahierten vorbestimmten Gebiet zu bestimmen; sowie
eine Anzeigesteuerungseinrichtung, die konfiguriert ist, eine Anzeigeeinrichtung (192) zu veranlassen, den durch die Bestimmungseinrichtung (194) bestimmten Krankheitstyp anzuzeigen.

2. Bildverarbeitungsvorrichtung nach Anspruch 1,
wobei die Bestimmungseinrichtung konfiguriert ist, den Krankheitstyp basierend auf einer Konnektivität zwischen benachbarten Pixeln der diskontinuierlichen Region zu bestimmen.

**3.** Bildverarbeitungsvorrichtung nach Anspruch 2,
wobei die Bestimmungseinrichtung konfiguriert ist, einen Typ Läsion an der diskontinuierlichen Region als den Krankheitstyp abhängig davon zu bestimmen, ob eine Breite einer Verteilung der diskontinuierlichen Region in der die Tiefenrichtung kreuzenden Richtung, wie auf dem Tomographiebild gesehen, größer als ein Schwellenwert ist oder nicht.

**4.** Bildverarbeitungsvorrichtung nach einem der Ansprüche 1 bis 3,
wobei die Anzeigesteuerungseinrichtung konfiguriert ist, die Anzeigeeinrichtung zu veranlassen, den Krankheitstyp basierend auf einem Parameter anzuzeigen, der die Diskontinuität der diskontinuierlichen Region angibt.

**5.** Bildverarbeitungsvorrichtung nach einem der Ansprüche 1 bis 4,
wobei die Anzeigesteuerungseinrichtung konfiguriert ist, die Anzeigeeinrichtung zu veranlassen, den Krankheitstyp basierend auf der Dicke und/oder der Breite der diskontinuierlichen Region, wie auf dem Tomographiebild gesehen, anzuzeigen.

**6.** Bildverarbeitungsvorrichtung nach einem der Ansprüche 1 bis 5,

wobei das vorbestimmte Gebiet ein depolarisiertes Gebiet ist, wo die Polarisation randomisiert ist, und
wobei die Anzeigesteuerungseinrichtung konfiguriert ist, die Anzeigeeinrichtung zu veranlassen, das depolarisierte Gebiet auf dem tomographischen Luminanzbild des Subjekts überlagert anzuzeigen, und die Anzeigeeinrichtung zu veranlassen, die die diskontinuierliche Region angebende Anzeigeform anzuzeigen, wobei die Anzeigeform auf dem tomographischen Luminanzbild überlagert ist.

**7.** Bildverarbeitungsvorrichtung nach einem der Ansprüche 1 bis 6,
wobei das polarisationsempfindliche Tomographiebild ein Tomographiebild ist, das basierend auf voneinander verschiedenen polarisierten Lichtarten erzeugt wird und das durch Aufteilen von Licht erfasst wird, in dem ein Rückstrahlenbündel, das von dem mit einem Messstrahlenbündel bestrahlten Subjekt zurückkehrt, mit einem dem Messstrahlenbündel entsprechenden Referenzstrahlenbündel kombiniert wird.

**8.** Bildverarbeitungsverfahren (S104) umfassend:

Erfassen eines mit polarisationsempfindlicher Tomographie gewonnenes Bilds, kurz: eines polarisationsempfindlichen Tomographiebilds, eines Subjekts;
Extrahieren eines vorbestimmten Gebiets aus dem erfassten polarisationsempfindlichen Tomographiebild;
Bestimmen eines Krankheitstyps basierend auf einem Zustand an einer diskontinuierlichen Region (605) im extrahierten vorbestimmten Gebiet; sowie
Veranlassen, dass eine Anzeigeeinrichtung den im Bestimmungsschritt bestimmten Krankheitstyp anzeigt (S105).

**9.** Bildverarbeitungsverfahren nach Anspruch 8,
wobei im Bestimmungsschritt der Krankheitstyp basierend auf einer Konnektivität zwischen benachbarten Pixeln der diskontinuierlichen Region bestimmt wird.

**10.** Bildverarbeitungsverfahren nach Anspruch 9, umfassend:
Bestimmen eines Typs Läsion an der diskontinuierlichen Region als der Krankheitstyp abhängig von einer Größe in Bezug auf den Schwellenwert von einer Breite einer Verteilung der diskontinuierlichen Region in einer eine Tiefenrichtung kreuzenden Richtung, wie auf dem Tomographiebild gesehen.

**11.** Bildverarbeitungsverfahren nach einem der Ansprüche 8 bis 10, umfassend:
Veranlassen, dass die Anzeigeeinrichtung den Krankheitstyp basierend auf einem Parameter anzeigt, der die Diskontinuität der diskontinuierlichen Region angibt.

**12.** Bildverarbeitungsverfahren nach einem der Ansprüche 8 bis 11, umfassend:
Veranlassen, dass die Anzeigeeinrichtung den Krankheitstyp basierend auf der Dicke und/oder der Breite der diskontinuierlichen Region, wie auf dem Tomographiebild gesehen, anzeigt.

**13.** Bildverarbeitungsverfahren nach einem der Ansprüche 8 bis 12,

wobei das vorbestimmte Gebiet ein depolarisiertes Gebiet ist, wo die Polarisation randomisiert ist, und das Verfahren umfasst: Veranlassen der Anzeigeeinrichtung, das depolarisierte Gebiet auf dem tomographischen Luminanzbild des Subjekts überlagert anzuzeigen, und weiterhin Veranlassen der Anzeigeeinrichtung, die die diskontinuierliche Region angebende Anzeigeform auf dem tomographischen Luminanzbild überlagert anzuzeigen.

14. Bildverarbeitungsverfahren nach einem der Ansprüche 8 bis 13,
wobei das polarisationsempfindliche Tomographiebild ein Tomographiebild ist, das basierend auf voneinander verschiedenen polarisierten Lichtarten erzeugt wird und das durch Aufteilen von Licht erfasst wird, in dem ein Strahlenbündel, das von dem mit einem Messstrahlenbündel bestrahlten Subjekt zurückkehrt, mit einem dem Messstrahlenbündel entsprechenden Referenzstrahlenbündel kombiniert wird.

15. Computerausführbares Programm, um einen Computer zu veranlassen, jeden Schritt des Bildverarbeitungsverfahrens nach einem der Ansprüche 8 bis 14 durchzuführen.

## Revendications

1. Appareil de traitement d'image, comprenant :

un moyen d'acquisition d'image tomographique (100) configuré pour acquérir une image tomographique sensible à la polarisation (600) d'un sujet (Er) ;
un moyen d'extraction configuré pour extraire une zone prédéterminée de l'image tomographique sensible à la polarisation acquise ;
un moyen de détermination (194) configuré pour déterminer un type de maladie sur la base d'un état au niveau d'une région discontinue (605) de la zone prédéterminée extraite ; et
un moyen de commande d'affichage configuré pour amener un moyen d'affichage (192) à afficher le type de maladie déterminé par le moyen de détermination (194).

2. Appareil de traitement d'image selon la revendication 1, dans lequel le moyen de détermination est configuré pour déterminer le type de maladie sur la base d'une connectivité entre des pixels adjacents de la région discontinue.

3. Appareil de traitement d'image selon la revendication 2, dans lequel le moyen de détermination est configuré pour déterminer un type de lésion au niveau de la région discontinue en tant que type de maladie conformément au fait qu'une largeur de distribution de la région discontinue dans la direction coupant la direction de profondeur, observée sur l'image tomographique, est, ou non, supérieure à un seuil.

4. Appareil de traitement d'image selon l'une quelconque des revendications 1 à 3, dans lequel le moyen de commande d'affichage est configuré pour amener le moyen d'affichage à afficher le type de maladie sur la base d'un paramètre indiquant la discontinuité de la région discontinue.

5. Appareil de traitement d'image selon l'une quelconque des revendications 1 à 4, dans lequel le moyen de commande d'affichage est configuré pour amener le moyen d'affichage à afficher le type de maladie sur la base d'au moins l'une de l'épaisseur et de la largeur de la région discontinue observées sur l'image tomographique.

6. Appareil de traitement d'image selon l'une quelconque des revendications 1 à 5,
dans lequel la zone prédéterminée est une zone dépolarisée dans laquelle la polarisation est randomisée, et
dans lequel le moyen de commande d'affichage est configuré pour amener le moyen d'affichage à afficher la zone dépolarisée superposée sur l'image de luminance tomographique du sujet et pour amener le moyen d'affichage à afficher la forme d'affichage indiquant la région discontinue avec la forme d'affichage superposée sur l'image de luminance tomographique.

7. Appareil de traitement d'image selon l'une quelconque des revendications 1 à 6, dans lequel l'image tomographique sensible à la polarisation est une image tomographique générée sur la base de lumières polarisées différentes les unes des autres et acquises par une division de lumière dans laquelle un faisceau de retour renvoyé à partir du sujet soumis à une exposition à un faisceau de mesure est combiné à un faisceau de référence correspondant au faisceau de mesure.

8.  Procédé de traitement d'image (104), consistant à :

    acquérir une image tomographique sensible à la polarisation d'un sujet ;
    extraire une zone prédéterminée de l'image tomographique sensible à la polarisation acquise ;
    déterminer un type de maladie sur la base d'un état au niveau d'une région discontinue (605) de la zone prédéterminée extraite ; et
    amener un moyen d'affichage à afficher (S105) le type de maladie déterminé à l'étape de détermination.

9.  Procédé de traitement d'image selon la revendication 8, dans lequel, à l'étape de détermination, le type de maladie est déterminé sur la base d'une connectivité entre des pixels adjacents de la région discontinue.

10. Procédé de traitement d'image selon la revendication 9, consistant à déterminer un type de lésion au niveau de la région discontinue en tant que type de maladie conformément à une taille par rapport à la valeur seuil de largeur de distribution de la région discontinue dans une direction coupant une direction de profondeur observée sur l'image tomographique.

11. Procédé de traitement d'image selon l'une quelconque des revendications 8 à 10, consistant à amener le moyen d'affichage à afficher le type de maladie sur la base d'un paramètre indiquant la discontinuité de la région discontinue.

12. Procédé de traitement d'image selon l'une quelconque des revendications 8 à 11, consistant à amener le moyen d'affichage à afficher le type de maladie sur la base d'au moins l'une de l'épaisseur et de la largeur de la région discontinue observées sur l'image tomographique.

13. Procédé de traitement d'image selon l'une quelconque des revendications 8 à 12,
    dans lequel la zone prédéterminée est une zone dépolarisée dans laquelle la polarisation est randomisée, et
    le procédé consistant à amener le moyen d'affichage à afficher la zone dépolarisée superposée sur l'image de luminance tomographique du sujet, et à amener en outre le moyen d'affichage à afficher la forme d'affichage indiquant la région discontinue superposée sur l'image de luminance tomographique.

14. Procédé de traitement d'image selon l'une quelconque des revendications 8 à 13, dans lequel l'image tomographique sensible à la polarisation est une image tomographique générée sur la base de lumières polarisées différentes les unes des autres et acquises par une division de lumière dans laquelle un faisceau renvoyé à partir du sujet soumis à une exposition à un faisceau de mesure est combiné à un faisceau de référence correspondant au faisceau de mesure.

15. Programme exécutable par ordinateur destiné à amener un ordinateur à exécuter chaque étape du procédé de traitement d'image selon l'une quelconque des revendications 8 à 14.

FIG. 1

EP 2 728 549 B1

# FIG. 3

```
        ┌─────────────┐
        │    START    │
        └──────┬──────┘
               │
               ▼
   ┌───────────────────────┐
   │  PERFORM ADJUSTMENT    │～S101
   └───────────┬───────────┘
               │
               ▼
   ┌───────────────────────┐
   │    CAPTURE  IMAGE      │～S102
   └───────────┬───────────┘
               │
               ▼
   ┌───────────────────────┐
   │   GENERATE  IMAGE      │～S103
   └───────────┬───────────┘
               │
               ▼
   ┌───────────────────────┐
   │   ANALYZE  IMAGE       │～S104
   └───────────┬───────────┘
               │
               ▼
   ┌───────────────────────┐
   │       OUTPUT          │～S105
   └───────────┬───────────┘
               │
               ▼
        ┌─────────────┐
        │     END     │
        └─────────────┘
```

FIG. 4

FIG. 5A

FIG. 5B

500

501

500

505

## FIG. 6A

600

601

602

## FIG. 6B

600

605

## FIG. 6C

610

611

612

## FIG. 6D

610

615

FIG. 7A

700

701

FIG. 7B

705

706

FIG. 7C

710

711

FIG. 7D

715

716

FIG. 7E

750

FIG. 8

FIG. 9A

FIG. 9B

900

902
901
903

900

905
903

FIG. 9C

FIG. 9D

910

912
914 913 911

910

914 913 915

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010122118 A1
- EP 2617351 A1
- EP 2574273 A1

**Non-patent literature cited in the description**

- **E. GOTZINGER et al.** *Opt. Express,* 2005, vol. 13, 10217